# EUROPEAN PATENT APPLICATION

(11) **EP 3 865 564 A1**
(43) Date of publication of application: **18.08.2021**
(21) Application number: 21155930.7
(22) Date of filing: 09.02.2021
(51) Int. Cl.: C12M 1/107, C12M 1/00, C02F 1/02

(54) **BIOMASS HYDROLYSIS PLANT**

(30) Priority: 17.02.2020 DK PA202070094
(71) Applicant: A J Inventing v/Anker Jarl Jacobsen, 2600 Glostrup (DK)
(72) Inventor: Jacobsen, Anker Jarl, 2600 Glostrup (DK)
(74) Representative: Zacco Denmark A/S

(57) **Abstract**

Herein is detailed a biomass hydrolysis plant (10) for continuous biomass hydrolysis of a flow of biomass, the biomass hydrolysis plant (10) comprising flowing biomass moving means, a continuous flow hydrolysis reactor (11), and a phase separator (12) for separating a flow of biomass comprising hydrolyzed biomass from a flow of exit gas; the continuous flow hydrolysis reactor (11) and phase separator (12) sequentially arranged and operatively connected for permitting a flow of biomass to traverse said biomass hydrolysis plant (10) for obtaining the flow of biomass comprising hydrolyzed biomass and the flow of exit gas.

## Description

### TECHNICAL FIELD

In the art of biomass hydrolysis plants there is detailed a biomass hydrolysis plant for continuous biomass hydrolysis having improved energy efficiency and utilization of by-product mass streams.

### BACKGROUND

It has been known for a considerable time that hydrolysis of biomass by heating, e.g. under pressure and using chemicals for promoting hydrolysis, will open biomass for improved production of biogas in a biogas plant, typically increasing the output from the biogas plant by 10-20%, when the increased energy cost of biomass hydrolysis at increased temperatures are factored in.

Typically, conventional biomass hydrolysis plants conventionally implement batch hydrolysis of biomass, which leads to reduced energy efficiency, as recirculation of the supplied heat is difficult between batches of biomass.

Also, conventional biomass hydrolysis plants implementing batch hydrolysis have little control of any gasses released at termination of the hydrolysis process, in particular including having difficulty of handling released ammonia (and other release gasses, e.g. H₂S).

For these reasons, the environmental authorities of Hjørring Kommune in Denmark recently order a forced closing of a batch biomass hydrolysis plant as secondary energy cost was uncontrolled and the control of released gasses was insufficient for staying within legal air pollution requirements.

DE 10 2011 120 888 B3 discloses a process for degradation of biogenic material, which process comprises carrying out thermo-mechanical pulping and recycling of food ingredients and other organic waste including fermentation residues in multistage system by homogenizing in mixing- and grinding system. In this process, the process units are arranged with considerable separation and with several further process units and reaction intermediates arranged therebetween.

The present inventor proposes a biomass hydrolysis plant suitable for continuous biomass hydrolysis, wherein in suitable embodiments of the biomass hydrolysis plant of the invention, supplied energy can be recycled. In particularly preferred embodiments, ammonia can be recovered from the biomass hydrolysis process as ammonium compositions suitable for use as fertilizers, whereby the only release product from the present plant becomes substantially pure water condensate. At the same time, high ammonia content biomass can be used directly in biogas plants after passage of the presently suggested biomass hydrolysis plants (10) without risking bacterial inhibition as ammonia will be effectively removed by the hydrolysis process.

### SUMMARY OF THE INVENTION

In accordance with the present invention there is herein detailed a biomass hydrolysis plant (10) for continuous biomass hydrolysis of a flow of biomass, the biomass hydrolysis plant (10) comprising flowing biomass moving means, a continuous flow hydrolysis reactor (11), and a phase separator (12) for separating a flow of biomass comprising hydrolyzed biomass from a flow of exit gas; the continuous flow hydrolysis reactor (11) and phase separator (12) sequentially arranged and operatively connected for permitting a flow of biomass to traverse the biomass hydrolysis plant (10) for obtaining the flow of biomass comprising hydrolyzed biomass and the flow of exit gas.

In an aspect and embodiment thereof, the continuous flow hydrolysis reactor (11) and phase separator (12) are sequentially arranged, and thereby coupled in direct fluid communication, on the biomass flowpath (1) and operatively connected for permitting the flow of biomass to traverse the biomass hydrolysis plant (10) for obtaining the flow of biomass comprising hydrolyzed biomass and the flow of exit gas.

In an aspect and embodiment thereof, a biomass hydrolysis plant (10) further comprising an exit gas scrubber section (20), the exit gas scrubber section (20) comprising a scrubber (21) arranged for scrubbing the flow of exit gas with a scrubbing solution for obtaining a resulting fertilizer solution and a flow of scrubbed gas as the flow of exit gas.

In an aspect and embodiment thereof, the scrubber (21) arranged for scrubbing the flow of exit gas is an adiabatic scrubber.

In an aspect and embodiment thereof, a biomass hydrolysis plant (10) further comprising a biomass pre-conversion section (30) for converting a flow of raw biomass to a flow of flowing biomass suitable for traversing the continuous flow hydrolysis reactor (11) as the flow of biomass.

In an aspect and embodiment thereof, a biomass hydrolysis plant (10) further comprising a hydrolysis water heating section (40) for providing heated and pressurized hydrolysis water to the flow of raw biomass and/or flow of flowing biomass prior to entry into the continuous flow hydrolysis reactor (11).

In an aspect and embodiment thereof, a biomass hydrolysis plant (10), wherein one or more of the flow of biomass, flow of biomass comprising hydrolyzed biomass, flow of exit gas, fertilizer solution, flow of scrubbed gas, flow or raw biomass, flow of flowing biomass and hydrolysis water can heat exchange with one or more flows or mass streams traversing said biomass hydrolysis plant (10). Preferably, such heat exchange leads to improved energy efficiency.

In an aspect and embodiment thereof, a biomass hydrolysis plant (10), wherein the flow of exit gas can heat exchange with the flow of biomass prior to entry of the flow of biomass into the continuous flow hydrolysis reactor (11).

In an aspect and embodiment thereof, a biomass hydrolysis plant (10), wherein the flow of exit gas can heat exchange with the flow of raw biomass.

In an aspect and embodiment thereof, a biomass hydrolysis plant (10), wherein the flow of biomass comprising hydrolyzed biomass and/or the fertilizing solution can heat exchange with the hydrolysis water.

In an aspect and embodiment thereof, a biomass hydrolysis plant (10), wherein the flow of biomass is submitted to hydrolysis during passage of the continuous flow hydrolysis reactor (11) at a hydrolysis temperature of from 120°C to 200°C at a corresponding hydrolysis pressure, for forming the flow of biomass comprising hydrolyzed biomass and a residual aqueous phase which are separated in the phase separator (12), such that the residual aqueous phase exits the phase separator (12) as the flow of exit gas.

### BRIEF DESCRIPTION OF THE DRAWINGS

- **Fig. 1**:: Biomass hydrolysis plant
- **Fig. 2:**: Biomass hydrolysis plant with scrubber
- **Fig. 3:**: Biomass hydrolysis plant with biomass feeder
- **Fig. 4:**: Biomass hydrolysis plant with heater
- **Fig. 5**:: Biomass hydrolysis plant - exemplary

### DETAILED DESCRIPTION

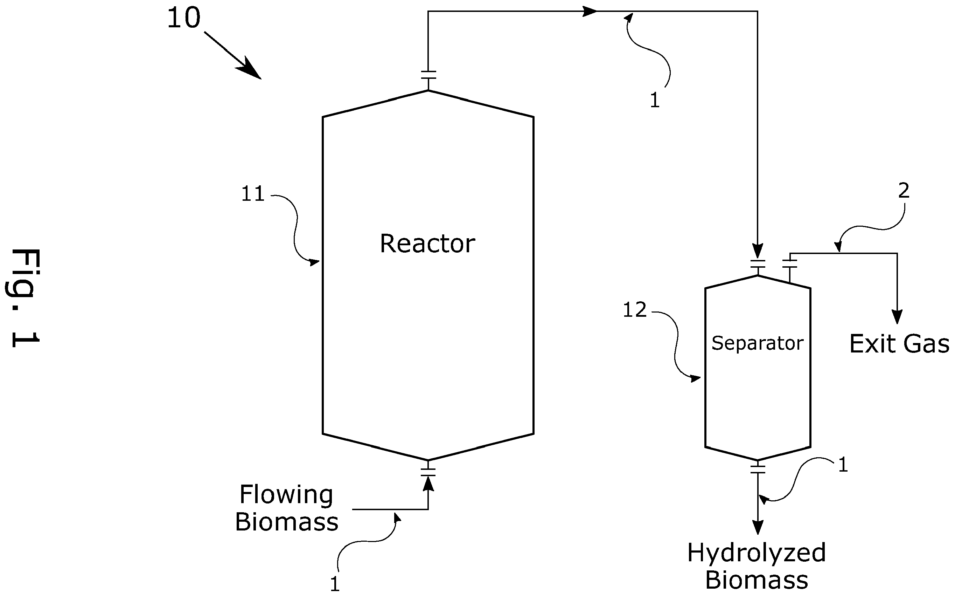

In accordance with the present invention, there is in Fig. 1 detailed an exemplary layout of a biomass hydrolysis plant (10) for continuous biomass hydrolysis comprising a biomass flowpath (1), comprising flowing biomass moving means, whereon a continuous flow hydrolysis reactor (11), and a phase separator (12) operatively connected to an exit gas flowpath (2) are sequentially arranged, and coupled in direct fluid communication, on the biomass flowpath (1).

The aspects and embodiments of the invention shown in the figures are exemplary of the invention, and it is understood that the skilled person based on the teachings disclosed herein will have knowledge to modify the herein disclosed biomass hydrolysis plant (10). In the Figures, like numbers correspond to like elements.

In the context of the present invention, biomass shall be understood as the dry matter fraction of biological matter, i.e. the fraction comprised of combustible matter and ashes.

For the purposes of the invention, raw biomass is the biomass supplied to the hydrolysis plant (10) from a source of biomass outside the hydrolysis plant (10). The raw biomass may comprise a certain amount of water, the inclusion of which is useful for the purposes of performing hydrolysis of the biomass in the biomass hydrolysis plant (10).

For the purpose of the present invention, particularly suitable sources of raw biomass are straw, dry manure such as e.g. birds' manure, in particular chicken manure, liquid manure (slurry), or preferably straw with manure, such as straw bedding (litter), deep bedding, deep litter, and/or muck.

For the purpose of the present invention, flowing biomass comprises raw biomass as detailed herein and hydrolysis water as detailed herein.

In some embodiments, the raw biomass is processed to obtain a size-reduced biomass having a higher surface area than the originating raw biomass. Hydrolysis water may or may not have been mixed to the raw biomass before size-reduction to obtain a flowing biomass. In some embodiments, the raw biomass is processed to obtain a size-reduced biomass suitable for forming a slurry with hydrolysis water, and in some embodiments, the slurry with hydrolysis water is a pumpable slurry.

In general, size-reduction of the raw biomass is valuable for increasing the output of the hydrolysis process and for improving the flow properties of the flowing biomass. However, for the purposes of the present invention, there are no particular limitations on the raw biomass or, where applicable, the size-reduced biomass, if a flowing biomass can be directly obtained from mixing raw biomass and hydrolysis water. In general and when used, size-reduction of the raw biomass for forming a flowing biomass of a size-reduced biomass will be a compromise between the energy cost of preprocessing the raw biomass to an improved size-reduced biomass and the additional yield from the hydrolysis from the improved size-reduction. Likewise, not all the raw biomass may become size-reduced during size-reduction without this having a significant influence on the outcome of the hydrolysis yield.

Depending on the source of biomass, the raw biomass may or may not be suitable for moving as a flowing biomass using flowing biomass moving means when received at the hydrolysis plant (10), and accordingly may or may not have to be converted to a flowing biomass prior to entering the continuous flow hydrolysis reactor (11).

As will be detailed herein, for performing a hydrolysis, hydrolysis water will normally be supplied to the raw biomass for obtaining a flowing biomass, but depending on the type of biomass, e.g. liquid manure, the raw biomass may already be a flowing biomass that can be supplied to the biomass hydrolysis plant (10).

In accordance with the present invention, hydrolysis water can be water from a low contamination source, such as e.g. ground water. In some embodiments thereof, the hydrolysis water may comprise hydrolysis-enhancing chemicals as is known in the art, examples of which include, e.g. alkaline and acid compounds, ammonia and compounds liberating ammonia.

It is preferable that the hydrolysis water is enriched in nitrogen, such as enriched in urea or the like. Suitable sources thereof are e.g. liquid manure and urine. In preferred embodiments of the present invention, the hydrolysis water is liquid manure, slurry or urine. In the most preferred embodiment, the hydrolysis water is liquid manure or slurry.

Depending on the desired purposes of the disclosed biomass hydrolysis plant (10), flowing biomass comprises 10-60% w/w biomass and 40-90% w/w hydrolysis water, including water present in the raw biomass. Preferably, flowing biomass comprises not more than 55% w/w biomass, not more than 50% w/w biomass, not more than 45% w/w biomass or not more than 40% w/w biomass based on total weight of biomass and water, with the balance being hydrolysis water. When the hydrolysis water is e.g. liquid manure, which comprises some biomass, the biomass content of the hydrolysis water is counted with the raw biomass and the aqueous phase of the liquid manure is counted with the hydrolysis water content together with the water present in the raw biomass.

Accordingly, there is herein detailed according to the present invention, a biomass hydrolysis plant (10) for continuous biomass hydrolysis of flowing biomass comprising: a biomass flowpath (1) comprising flowing biomass moving means; a continuous flow hydrolysis reactor (11); and a phase separator (12) operatively connected to an exit gas flowpath (2); the continuous flow hydrolysis reactor (11) and phase separator (12) sequentially arranged on the biomass flowpath (1) such that flowing biomass can be:
- submitted to hydrolysis during passage of the continuous flow hydrolysis reactor (11); and
- separated from the residual aqueous phase in the phase separator (12), such that the residual aqueous phase exits the phase separator (12) as an exit gas along the exit gas flowpath (2), and the biomass comprising hydrolyzed biomass exits the phase separator (12) along the biomass flowpath (1).

In an embodiment thereof, hydrolysis for forming a hydrolyzed biomass and a residual aqueous phase is at a hydrolysis temperature of from 120°C to 200°C at a corresponding hydrolysis pressure.

In the plant (10), flowing biomass as a continuous mass flow stream is moved along the biomass flowpath (1) by the flowing biomass moving means comprised in the biomass flowpath (1). Depending on the ratio of raw biomass to hydrolysis water, the flowing biomass moving means can be pumps, e.g. mono and hose pumps, snails, screws or combinations thereof, or combinations thereof with locks or sluices for preventing backflow of the flowing biomass and securing maintenance of an achieved pressure, in particular for securing an achieved corresponding hydrolysis pressure.

In some embodiments, a sequence of flowing biomass moving means and locks are arranged on the biomass flowpath (1) prior to the continuous flow hydrolysis reactor (11) for increasing a pressure of the flowing biomass, preferably for increasing a pressure of the flowing biomass to the aforementioned corresponding hydrolysis pressure, and for increasing the content of dry material.

In accordance with the invention, the continuous flow hydrolysis reactor (11) is suitable for holding under pressure and increased temperature a flowing biomass for a given holding time, sufficiently to hydrolyze the biomass to a given target, such as until 50% w/w of the biomass has been hydrolyzed, until 60% w/w, until 70% w/w, until 80% w/w, until 90% w/w, or until 95% w/w of the biomass has been hydrolyzed.

As, in general, the efficacy of the subsequent biogas (or other biofuels) production is enhanced by the efficacy of the hydrolysis process, long holding times favor increased hydrolysis; however, at the cost of increased production energy expenditure and larger plants. Accordingly, in some embodiments the holding time is 2 hours, 1.5 hours, 1 hour, preferably from 0.25 hours to 0.75 hours, more preferably 0.5 hours.

In accordance with the invention, hydrolysis in the continuous flow hydrolysis reactor (11) occurs at a hydrolysis temperature from 120°C to 200°C at a corresponding hydrolysis pressure. Preferably, the hydrolysis temperature is from 125°C to 195°C, from 120°C to 190°C, from 135°C to 185°C, from 140°C to 180°C, from 145°C to 175°C, from 150°C to 170°C, from 155°C to 165°C, or 160°C.

It is possible to select higher hydrolysis temperatures than 200°C, but this is of less economic value due to the increased cost of heating, making any resulting biofuel obtained from the hydrolyzed biomass less valuable. Further, and as will be detailed below, ammonia is a valuable by-product of the present biomass hydrolysis plant (10) and at temperatures over 200°C, the ammonia-water shift reaction can further lower the economic value of the byproduct.

A corresponding hydrolysis pressure is a pressure at which pressure and corresponding hydrolysis temperature, water does not boil. Depending on the design of the continuous flow hydrolysis reactor (11), the flowing biomass will normally be hottest during holding which sets an upper minimum pressure for the corresponding hydrolysis pressure, which must at least be large enough to maintain the aqueous phase liquid. Such corresponding pressures and temperatures are well known tabular values, e.g. water at 120°C requires a corresponding pressure of 2 bar to remain liquid, whereas water at 200°C requires a corresponding pressure of 15.5 bar to stay liquid. In some embodiments, and for reasons of safety, the corresponding hydrolysis pressure can be higher by a safety factor than the upper minimum pressure for maintaining the aqueous phase liquid at a selected hydrolysis temperature. However, normally the conditions of operation will be equilibrium conditions for temperature and pressure.

In accordance with the invention, heating of the flowing biomass may take place prior to entering the continuous flow hydrolysis reactor (11), it is however preferable that heating takes place within the reactor (11) itself. In some embodiments, the continuous flow hydrolysis reactor (11) comprises a heating section and a holding section, which allows a more versatile control of heating. Nevertheless, irrespective of the heating design of the continuous flow hydrolysis reactor (11), the holding time will correspond to the time spent in the reactor (11) by the biomass, from entry into the reactor (11) to its exit.

It is an advantage of the present invention that when the continuous flow hydrolysis reactor (11) is operated under conditions suitable for holding under pressure and increased temperature a flowing biomass for a given hydrolysis time, sufficiently to hydrolyze the biomass in accordance with the above, the reactor (11) can be designed as a plug-flow reactor, wherein mixing can be ignored between plugs in the direction of the biomass flow.

This allows for a simplified design of the continuous flow hydrolysis reactor (11), which in embodiments of the plug-flow reactor can be designed as a tubular reactor. In further embodiments of the plug-flow reactor, the reactor (11) may comprise means for preventing canal formation, such as e.g. flow obstacles or passive mixers.

In accordance with the present disclosure, the biomass comprising the hydrolyzed biomass is separated from the residual aqueous phase in the phase separator (12). Thereby the residual aqueous phase exits the phase separator (12) as a gas along the the exit gas flowpath (2) operatively connected to the phase separator (12), and the biomass comprising the hydrolyzed biomass exits the phase separator (12) along the biomass flowpath (1).

While it is possible to perform a solid-liquid separation in the phase separator (12), this is in general not preferable in relation to processes for hydrolyzing biomass. Rather, increased biomass disruption can be achieved, with resulting increased availability of digestible biomass, using rapid pressure decompression, such as e.g. flash expansion or steam explosion techniques.

In the present disclosure, the phase separator (12) preferably in all embodiments is a rapid pressure-decompression phase separator (12), such as a flash expansion reactor. For optimal biomass disruption, a large pressure drop over the rapid pressure-decompression phase separator (12) is desirable; however, it is in general not contemplated to provide a vacuum with the separator (12). Rather, and for use with the below detailed second aspect and embodiment of the invention, it is preferred that the resulting release pressure in the phase separator (12) is atmospheric pressure.

The biomass comprising hydrolyzed biomass will comprise any non-volatile ashes present in the flowing biomass, whereas the exit gas will comprise the volatile inorganic biomass fraction, primarily ammonia, and some amounts of atmospheric air originally comprised in the raw biomass and in the hydrolysis water, however, the exit gas will predominantly be steam at the release pressure of the phase separator (12) .

As the biomass comprising hydrolyzed biomass and the exit gas, in the preferred embodiments of the present invention, leave the phase separator (12) at atmospheric release pressure, the two separated mass fractions will still comprise significant energy in the form of heat at 100°C, the use of which energy is one subject of the present invention.

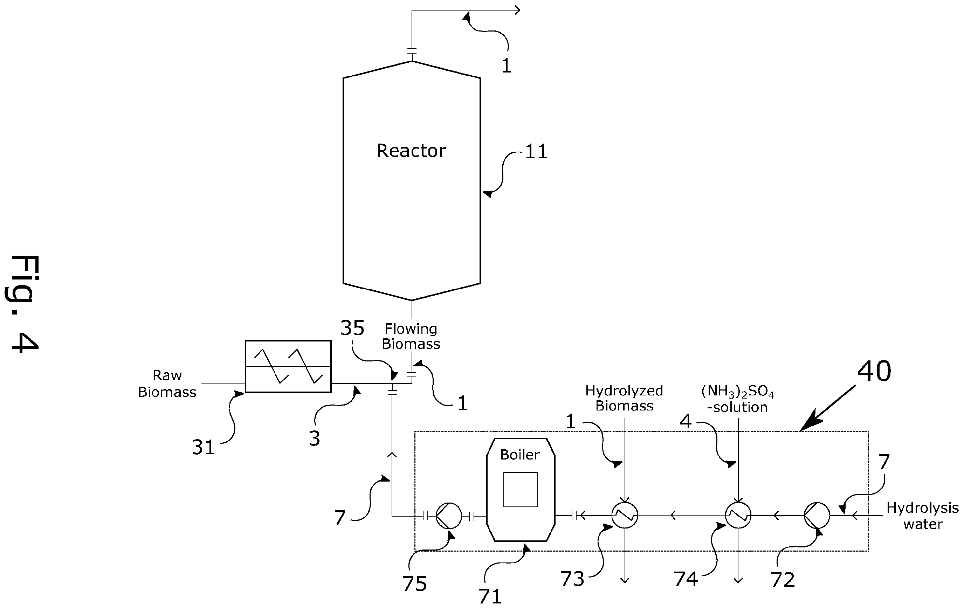

While the biomass comprising hydrolyzed biomass can be fed to a biogas plant as is, it is preferable, in accordance with embodiments of the present biomass hydrolysis plant (10), that the biomass comprising hydrolyzed biomass can heat exchange, at least partially, with other mass streams traversing the present biomass hydrolysis plant (10) for improved energy efficiency, such as e.g. heat exchange with the hydrolysis water (c.f. Fig. 4).

Normally, the exit gas will comprise a significant amount of ammonia liberated from the biomass and the hydrolysis water, e.g. when the hydrolysis water is liquid manure, and concentrated in the residual aqueous phase during hydrolysis. This ammonia can, due to the continuous nature of hydrolysis process, be obtained and recovered in a simple manner by inflow scrubbing with a scrubbing solution comprising an aqueous solution of either a nitric, a phosphoric or a sulfuric acid, for forming aqueous solutions of ammonium of a respective acid suitable for use as fertilizer solutions.

Thereby an economically valuable byproduct of the biomass hydrolysis is obtained, which under the right conditions, by itself, can be marketed at a value higher than the operating costs of the present biomass hydrolysis plant (10). However, at the same time a significant environmental problem, the uncontrolled release of ammonia to the environment, is eliminated, and recycling of bioavailable ammonia made possible.

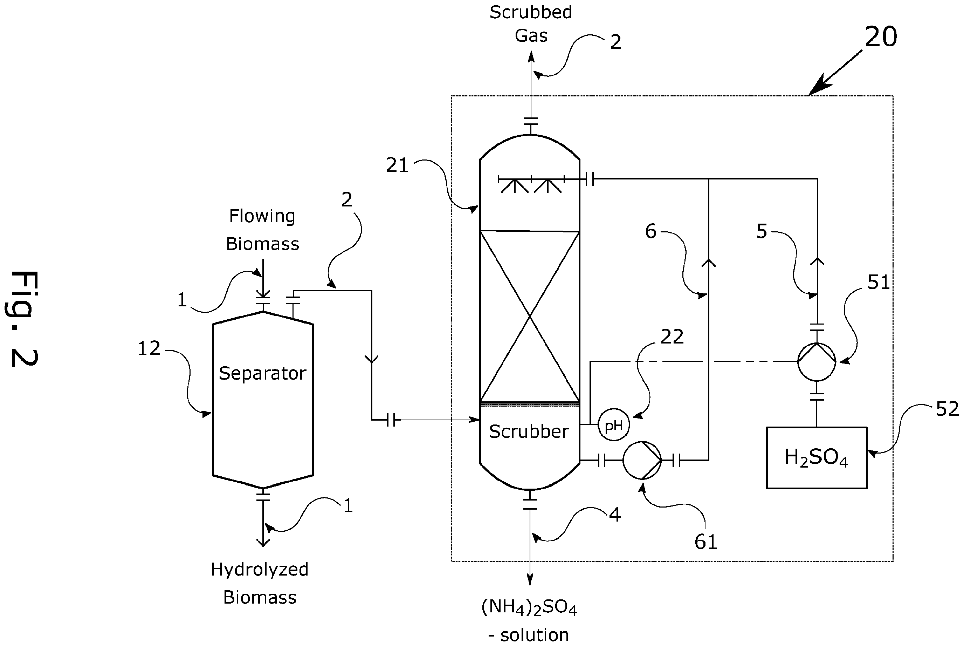

In accordance with the present invention in a second aspect and embodiment of the biomass hydrolysis plant (10), exemplarily shown in Fig. 2, part of the excess energy comprised in the exit gas is used for obtaining a fertilizer by scrubbing the exit gas received from the phase separator (12) with a scrubbing solution comprising an aqueous solution of either a nitric, a phosphoric or a sulfuric acid. In Fig. 2, this aspect and embodiment of the biomass hydrolysis plant (10) is detailed using sulfuric acid, however this is, while a preferred embodiment, merely for the purposes of illustrating the present invention.

In accordance with the second aspect and embodiment of the invention, there is herein disclosed the biomass hydrolysis plant (10) according to the above embodiments further comprising an exit gas scrubber section (20) for obtaining a resulting fertilizer solution, preferably a resulting fertilizer solution comprising ammonium.

In accordance with the invention for the biomass hydrolysis plant (10) comprising the exit gas scrubber section (20), the exit gas scrubber section (20) comprises a scrubber (21) arranged for scrubbing the flow of exit gas with a scrubbing solution to obtain a resulting fertilizer solution and a flow of scrubbed gas as the flow of exit gas, preferably obtaining a flow of scrubbed gas as the flow of exit gas for heat exchange with the aforementioned flow of biomass.

In accordance with the invention, the scrubber (21) is arranged on the exit gas flowpath (2) for scrubbing the exit gas with a scrubbing solution for obtaining a resulting fertilizer solution. Further, in accordance with the invention, the scrubber (21) preferably is operatively connected to a fertilizer solution exit-flowpath (4) for withdrawing the resulting fertilizer solution from the biomass hydrolysis plant (10).

In accordance with the invention, the exit gas scrubber section (20) comprises a scrubber (21) arranged on the exit gas flowpath (2) for scrubbing the exit gas with a scrubbing solution and operatively connected to a fertilizer solution exit-flowpath (4). Preferably, the scrubber (21) is a counter-flow scrubber (21), and even more preferably, the counter-flow scrubber (21) is an adiabatic counter-flow scrubber (21).

According to the use in the preferred embodiment of the invention, c.f. Fig. 2, the exit gas from the phase separator (12) enters and exits the scrubber (21) along the exit gas flowpath (2), exiting as a scrubbed gas.

It is preferable that the scrubber (21) is designed such that essentially complete absorption of any ammonia present in the exit gas at entry into the scrubber (21) takes place during the passage of the exit gas through the scrubber. In some embodiments of the invention, the scrubbed gas does not comprise more than 100 ppm of ammonia, not more than 75 ppm of ammonia, not more than 50 ppm of ammonia, not more than 25 ppm of ammonia, or more preferable, not more than 10 ppm of ammonia.

Generally, the scrubbed gas will not comprise water-soluble gasses below minor amounts. Rather, when, as preferred, the resulting release pressure in the phase separator (12) is atmospheric pressure, the scrubbed gas will primarily be steam at 100°C and any residual atmospheric gas as detailed above.

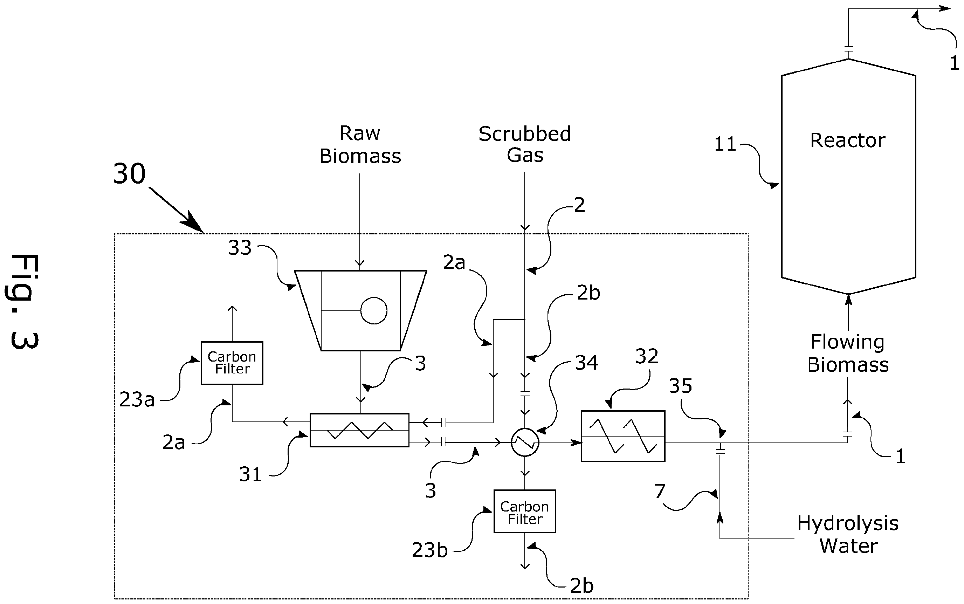

In accordance with embodiments of the present biomass hydrolysis plant (10), the scrubbed gas may heat exchange with other mass streams traversing the present biomass hydrolysis plant (10) for improved energy efficiency, such as e.g. heat exchange with the hydrolysis water. As will be detailed (c.f. Fig 3), the scrubbed gas is suitably used for heat exchange with biomass or hydrolysis water prior to entry of flowing biomass into the continuous flow hydrolysis reactor (11), thereby providing improved energy efficiency to the disclosed biomass hydrolysis plant (10).

According to the invention, the resulting fertilizer solution exits the scrubber (21) along the fertilizer solution exit-flowpath (4). The resulting fertilizer solution preferably exits the scrubber (21) as a concentrated fertilizer solution at the relevant exit temperature from the scrubber (21). In some embodiments, the resulting fertilizer solution exits the scrubber (21) as a saturated solution at the relevant exit temperature from the scrubber (21). In the preferred embodiments, wherein the scrubber (21) is an adiabatic counter-flow scrubber (21), the relevant exit temperature is liquid water at 100°C.

For operating the scrubber (21) in accordance with the invention, scrubbing solution is provided along a scrubbing solution flowpath (5) operatively connected to the scrubber (21), preferably provided to the scrubber (21) under pressure using a scrubber solution pump (51) from a scrubber solution reservoir (52).

In some embodiments of the invention (not shown), more than one scrubber solution reservoir (52) can be comprised, each respective scrubber solution reservoir (52) holding a respective scrubber solution, e.g. a first reservoir holding sulfuric acid, a second reservoir holding nitric acid, and/or a third reservoir holding a phosphoric acid, or e.g. serving as a secondary reservoir for a respective scrubber solution such that process continuation is not hindered if a respective first reservoir is emptied during biomass hydrolysis plant (10) operation. The skilled person will know how to employ a plurality of scrubber solution pumps (51) and valves for providing respective scrubber solutions from a respective reservoir (52) to the scrubber along respective or a common scrubber solution flowpath (5).

In preferred embodiments of the invention, the scrubber (21) comprises a pH-meter (22) arranged for determining the resulting pH of the resulting fertilizer solution, the pH-meter (22) operatively connected to the scrubber solution pump (51) for adjusting an amount of scrubber solution reaching the scrubber (21) along the scrubber solution flowpath (5).

In preferred embodiments of the biomass hydrolysis plant (10), the scrubber (21) is operatively connected to a fertilizer solution recirculation-flowpath (6) comprising recirculation pumping means (61), for concentration recirculation of the fertilizer solution. Thereby it will be possible to increase the concentration of the resulting fertilizing solution as it circulates the scrubber (21), as is known in the art. In this manner, both concentrated and saturated fertilizer solutions can be obtained at the relevant exit temperature for the fertilizer solutions from the scrubber (21).

In general, the resulting fertilizer solutions are suitable for use in agriculture as solutions; hence, in general, it will not be necessary to concentrate the resulting fertilizer solutions beyond a necessary concentration level for use in agriculture. E.g., ammonium sulfate is suitable for agricultural use as a 40% w/w concentration aqueous solution of ammonium sulfate, which concentration is below the saturation concentration of ammonium sulfate in water at 100°C. When e.g. this commercial concentration level is satisfactory, concentration recirculation is only operated to this level. However, in some cases it can be of commercial value to concentrate the resulting fertilizer solutions further at the relevant exit temperature, such that a fertilizer precipitate will form at lower temperatures than the relevant exit temperature. This enhances transportability of the resulting fertilizer solutions and precipitates. In embodiments (not shown), where a fertilizer precipitate is desired, the fertilizer solution exit-flowpath (4) may comprise a crystallizer downstream of the scrubber, such as e.g. a clarifier or the like.

In accordance with embodiments of the present biomass hydrolysis plant (10), the resulting fertilizer solutions may heat exchange with other mass streams traversing the present biomass hydrolysis plant (10) for improved energy efficiency, such as e.g. heat exchange with the hydrolysis water (c.f. Fig. 4). Where heat exchange with other traversing mass streams is contemplated, it is preferable, not to concentrate the resulting fertilizer solutions to a concentration higher than a saturation concentration at a temperature of exit from a heat exchange with another traversing mass stream. Thereby unwanted or premature crystallization during heat exchange is avoided.

In accordance with an aspect and embodiment of the present biomass hydrolysis plant (10) (c.f. Fig. 3), there is herein detailed the biomass hydrolysis plant (10) further comprising a biomass pre-conversion section (30) for converting a flow of raw biomass to a flow of flowing biomass suitable for traversing the continuous flow hydrolysis reactor (11).

In accordance with the invention, it is contemplated that raw biomass traverses the biomass pre-conversion section (30) exiting as flowing biomass that is suitable for flowing in the continuous flow hydrolysis reactor (11) during biomass hydrolysis, if not already received at the biomass hydrolysis plant (10) as flowing biomass. To this purpose, biomass can be moved and, optionally size-reduced, and hydrolysis water can be mixed with the biomass traversing the biomass pre-conversion section (30) for adjusting the water content of the traversing biomass to a water content suitable for use during hydrolysis.

In accordance with the invention, the biomass pre-conversion section (30) comprises a biomass conversion flowpath (3) whereon there is arranged at least a first feeder (31). In preferred embodiments, the first feeder (31) is a screw feeder. In some embodiments, the first feeder (31) further can serve as biomass size-reduction means, if the raw biomass reaching the first feeder (31) is too large for forming a flowing biomass suitable for use in the continuous flow hydrolysis reactor (11).

In accordance with a preferred embodiment of the biomass pre-conversion section (30), at least a second feeder (32) is arranged on the biomass conversion flowpath (3) subsequent to the first feeder (31). In preferred embodiments, the second feeder (32) is a screw feeder. In some embodiments, the second feeder (32) further can serve as biomass size-reduction means, if the raw biomass reaching the second feeder (32) is too large for forming a flowing biomass suitable for use in the continuous flow hydrolysis reactor (11) .

It is contemplated that the first feeder (31) or the first feeder (31) and the at least a second feeder (32) can form the aforementioned flowing biomass moving means.

In accordance with a preferred embodiment of the biomass pre-conversion section (30), locks or sluices are arranged on the biomass conversion flowpath (3) for preventing biomass backflow and preserving a resulting pressure obtained by passage of a feeder (31,32).

In accordance with a preferred embodiment of the biomass pre-conversion section (30), a mixing point (35) for biomass and hydrolysis water is arranged on biomass conversion flowpath (3) subsequent to the first feeder (31). In embodiments, the mixing point (35) is arranged subsequent to both the first feeder (31) and the second feeder (32). In embodiments, the mixing point (35) is operatively connected to a hydrolysis water flowpath (7). In embodiments, heated hydrolysis water is mixed with the traversing biomass for obtaining a heated flowing biomass. In preferred embodiments, the resulting heated flowing biomass has a temperature corresponding to the hydrolysis temperature during hydrolysis of the flowing biomass in the continuous flow hydrolysis reactor (11).

In accordance with a preferred embodiment of the biomass pre-conversion section (30), a hopper (33) is arranged prior to the first feeder (31). In preferred embodiments, the hopper (33) comprises locks or sluices.

In accordance with a preferred embodiment of the invention, scrubbed gas can heat exchange with raw biomass along the biomass conversion flowpath (3).

In an embodiment thereof, scrubbed gas is supplied along a second branch (2b) of the exit gas flowpath (2) to a heat exchanger (34) arranged for indirect heat exchange on the biomass conversion flowpath (3) before the mixing point (35), preferably before any second or further feeder (32).

Most preferably, however, the scrubbed gas is supplied along a first branch (2a) of the exit gas flowpath (2) to the first feeder (31) for direct heat exchange with the raw biomass such that scrubbed gas traverses the first feeder (31) in contact with the raw biomass, preferably traverses the first feeder (31) in countercurrent to the raw biomass. Thereby the water in the scrubbed gas is taken up by the raw biomass while the biomass is heated, and recirculated, thus reducing the need for hydrolysis water.

Irrespective of whether the scrubbed gas exits the biomass hydrolysis plant (10) along the exit gas flowpath (2) or along the respective first or second branches (2a,2b), it is preferable to provide a carbon filter (23a,23b) prior to release of the scrubbed gas to the atmosphere for removing any trace pollutants, such as e.g. H₂S or the like.

In accordance with an aspect and embodiment of the present biomass hydrolysis plant (10) (c.f. Fig. 4), there is herein detailed the biomass hydrolysis plant (10) further comprising a hydrolysis water heating section (40) for providing heated and pressurized hydrolysis water to a flow of raw or flowing biomass.

It is a purpose in embodiments of in the present invention of providing hydrolysis water to a raw biomass for obtaining a flowing biomass, but also of valorizing ammonia-rich liquids, such as liquid manure, in the biomass hydrolysis plant (10). This can be done by pre-mixing raw biomass with hydrolysis water prior to traversing the aforementioned first feeder (31) arranged on the aforementioned biomass conversion flowpath (3) and subsequently heated. However, as one or more heaters arranged on the biomass conversion flowpath (3) are less suitable for suitably heating flowing biomass for use in the biomass hydrolysis plant (10) of the invention, this leaves a problem of obtaining a sufficiently heated flowing biomass prior to entry into the continuous flow hydrolysis reactor (11), a problem, which is herein solved by providing a separate hydrolysis water heating section (40) comprising hydrolysis water heating means (71), e.g. a boiler (71).

In accordance with the invention, the hydrolysis water heating section (40) comprises a hydrolysis water flowpath (7) operatively connected to a mixing point (35) for biomass and hydrolysis water, whereon is arranged hydrolysis water heating means (71) and at least one hydrolysis water pump (72, 75) for raising a hydrolysis water pressure, preferably for raising a hydrolysis water pressure at least to a corresponding hydrolysis pressure. In embodiments, the at least one hydrolysis water pump forms part of the flowing biomass moving means.

In preferred embodiments thereof, there is further arranged at least one heat exchanger (73,74) on the hydrolysis water flowpath (7) prior to the hydrolysis water heating means (71), for permitting heat exchange with respectively hydrolyzed biomass (73) or resulting fertilizer solution (74). Thereby residual heat comprised in a flow of hydrolyzed biomass, respectively in a flow of resulting fertilizer solution, can be returned to the biomass hydrolysis plant (10) .

In embodiments of the invention, it is contemplated that hydrolysis water is heated at atmospheric pressure and supplied under pressure to the aforementioned mixing point (35). In embodiments of thereof, it is preferred that hydrolysis water is boiled at atmospheric pressure and supplied as steam to the aforementioned mixing point (35), preferably supplied as steam under pressure to the aforementioned mixing point (35).

In embodiments of the invention, it is contemplated that hydrolysis water is heated and pressurized in the hydrolysis water heating section (40) to a resulting hydrolysis water temperature and pressure at least corresponding to the aforementioned hydrolysis temperature and corresponding hydrolysis pressure. Thereby raw or flowing biomass, which is mixed with provided heated hydrolysis water at the mixing point (35), obtains a resulting temperature and resulting pressure, which is suitable for entry into the aforementioned biomass flowpath (1).

In embodiments, the resulting heated hydrolysis water temperature and pressure can be higher than a desired hydrolysis temperature and corresponding desired hydrolysis pressure, thus permitting any raw or flowing biomass admixed to the heated and pressurized hydrolysis water at the mixing point (35) to become heated to the desired hydrolysis temperature prior to entry into the continuous flow hydrolysis reactor (11).

### EXAMPLE

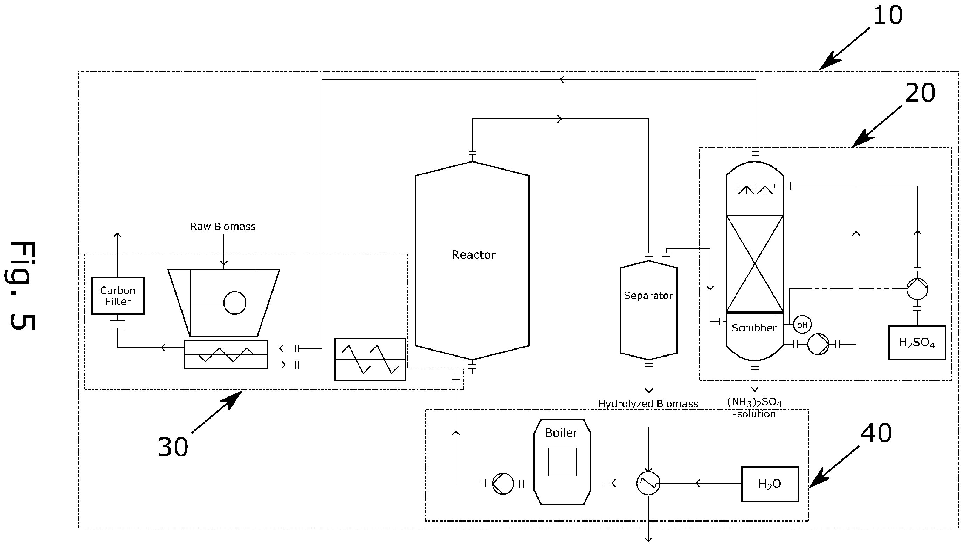

In an exemplary calculation based on a biomass hydrolysis plant (50) (c.f. Fig. 5) comprising a scrubber section (20), a biomass pre-conversion section (30) and a hydrolysis water heating section (40), the benefits of the present invention were examined.

Deep litter was selected as raw biomass and flowing biomass comprised 40% w/w dry matter. Conventional bio-methane production provides 80 m³ of methane/ metric ton of dry litter, which can be increased by 32 m³ of methane/ metric ton raw biomass following hydrolysis of the deep litter at 160°C for 30 min.

A conventional biomass hydrolysis plant without continuous hydrolysis and using a hydrolysis temperature of 160°C was compared with a biomass hydrolysis plant (10) of the invention comprising a continuous flow hydrolysis reactor (11), wherein scrubbed gas was fed along exit gas flowpath (2) into the at least a first feeder (31) at 100°C.

Heating deep litter from 20°C to 160°C requires 140 Mcal, requiring about 18 m³ of methane/ metric ton of dry litter, whereas recycling scrubbed gas steam recovers 60 Mcal, meaning that the plant (50) of the example requires only 80 Meal of energy for operation corresponding to about 10 m³ of methane, providing a net benefit from hydrolysis of about 22 m³ of methane compared to the 14 m³ of methane of the prior art plant.

At the same time, following the preferred embodiments of the present plant (10) comprising a scrubbing section (30) further economic benefit is obtained from recovering released ammonia as a resulting fertilizer solution. Implementing heat exchange of hydrolyzed biomass and/or resulting fertilizer solution will further increase the net energy benefit of the biomass hydrolysis plant (50) of the invention.

### CLOSING COMMENTS

The term "comprising" as used in the claims does not exclude other elements or steps. The term "a" or "an" as used in the claims does not exclude a plurality. A single processor or other unit may fulfill the functions of several means recited in the claims. A reference sign used in a claim shall not be construed as limiting the scope. Although the present invention has been described in detail for purpose of illustration, it is understood that such detail is solely for that purpose, and variations can be made therein by those skilled in the art without departing from the scope of the invention.

## Claims

1. A biomass hydrolysis plant (10) for continuous biomass hydrolysis of a flow of biomass, the biomass hydrolysis plant (10) comprising flowing biomass moving means, a continuous flow hydrolysis reactor (11), and a phase separator (12) for separating a flow of biomass comprising hydrolyzed biomass from a flow of exit gas; the continuous flow hydrolysis reactor (11) and phase separator (12) sequentially arranged and operatively connected for permitting a flow of biomass to traverse said biomass hydrolysis plant (10) for obtaining said flow of biomass comprising hydrolyzed biomass and said flow of exit gas.

2. A biomass hydrolysis plant (10), according to claim 1, wherein said continuous flow hydrolysis reactor (11) and phase separator (12) are sequentially arranged, and thereby coupled in direct fluid communication, on a biomass flowpath (1).

3. A biomass hydrolysis plant (10), according to claim 1 or claim 2, further comprising an exit gas scrubber section (20), said exit gas scrubber section (20) comprising a scrubber (21) arranged for scrubbing said flow of exit gas with a scrubbing solution for obtaining a resulting fertilizer solution and a flow of scrubbed gas as said flow of exit gas.

4. A biomass hydrolysis plant (10) according to any of the previous claims, wherein said scrubber (21) arranged for scrubbing said flow of exit gas is an adiabatic scrubber.

5. A biomass hydrolysis plant (10) according to any of the previous claims, further comprising a biomass pre-conversion section (30) for converting a flow of raw biomass to a flow of flowing biomass suitable for traversing the continuous flow hydrolysis reactor (11) as said flow of biomass.

6. A biomass hydrolysis plant (10) according to any of the previous claims further comprising a hydrolysis water heating section (40) for providing heated and pressurized hydrolysis water to said flow of raw biomass and/or flow of flowing biomass prior to entry into said continuous flow hydrolysis reactor (11).

7. A biomass hydrolysis plant (10) according to any of the previous claims, wherein one or more of said flow of biomass, said flow of biomass comprising hydrolyzed biomass, said flow of exit gas, said fertilizer solution, said flow of scrubbed gas, said flow or raw biomass, said flow of flowing biomass and said hydrolysis water are heat exchanged with one or more flows or mass streams traversing said biomass hydrolysis plant (10).

8. A biomass hydrolysis plant (10) according to any of the previous claims, wherein said flow of exit gas is heat exchanged with said flow of biomass prior to entry of said flow of biomass into said continuous flow hydrolysis reactor (11).

9. A biomass hydrolysis plant (10) according to any of the previous claims, wherein said flow of exit gas is heat exchanged with said flow of raw biomass.

10. A biomass hydrolysis plant (10) according to any of the previous claims, wherein one or more of said flow of biomass comprising hydrolyzed biomass and said fertilizing solution are heat exchanged with said hydrolysis water.

11. A biomass hydrolysis plant (10) according to any of the previous claims, wherein said flow of biomass is submitted to hydrolysis during passage of said continuous flow hydrolysis reactor (11) at a hydrolysis temperature of from 120°C to 200°C at a corresponding hydrolysis pressure, for forming said flow of biomass comprising hydrolyzed biomass and a residual aqueous phase which are separated in said phase separator (12), such that said residual aqueous phase exits said phase separator (12) as said flow of exit gas.
